# EUROPÄISCHE PATENTSCHRIFT

(11) **EP 1 475 079 B1**
(45) Veröffentlichungstag und Bekanntmachung des Hinweises auf die Patenterteilung: **14.06.2006**
(21) Anmeldenummer: 04007373.6
(22) Anmeldetag: 26.03.2004
(51) Int. Cl.: C08F 6/12, C08F 220/58, C08F 226/00

(54) **Stabile Polymer-Konzentrate sowie Verfahren zu ihrer Herstellung**
Stable polymer concentrate and process for its preparation
Concentrés de polymères et leur procédé de préparation

(30) Priorität: 03.04.2003 DE 10315182
(43) Veröffentlichungstag der Anmeldung: 10.11.2004
(73) Patentinhaber: Clariant Produkte (Deutschland) GmbH, 65929 Frankfurt am Main (DE)
(72) Erfinder: Löffler, Matthias, Dr., 65527 Niedernhausen (DE); Morschhäuser, Roman, Dr., 55122 Mainz (DE)

(56) Entgegenhaltungen:
- EP-A- 1 028 129
- EP-A- 1 116 733
- WO-A-02/44231

## Beschreibung

Die vorliegende Erfindung betrifft ein Verfahren zur Herstellung von Konzentraten aus Copolymeren auf Basis von Acryloyldimethyltaurinsäure bzw. deren Salzen und linearen und/oder cyclischen N-Vinylcarbonsäureamiden.

In der WO 02/44231 wird eine neue Klasse von Polymeren auf Basis von Acryloyldimethyltaurinsäure bzw. deren Salzen beschrieben. Diese Polymere decken breite anwendungstechnische Eigenschaften ab und können als Verdicker, Konsistenzgeber, Emulgator, Dispergator, Gleitmittel, Conditioner und/oder Stabilisator in kosmetischen, dermatologischen und pharmazeutischen Mitteln eingesetzt werden.

Die bevorzugt durch Fällungspolymerisation hergestellten Copolymere auf Basis von Acryloyldimethyltaurinsäure bzw. deren Salzen entsprechend dem Stand der Technik sind pulverförmige Substanzen mit dadurch resultierenden anwendungstechnischen Nachteilen. Pulverförmige Substanzen bergen prinzipiell Staubexplosionsgefahr, ferner ist die Lagerstabilität der Pulver durch Hygroskopie beeinträchtigt.

Zur Verarbeitung bzw. Verwendung der pulverförmigen Produkte ist der Lösevorgang (bevorzugt werden die Polymere in wässrige Medien eingearbeitet) meistens sehr zeitaufwendig. Der Lösevorgang der pulverförmigen Produkte kann, je nach Ansatzgröße, eine Stunde und mehr betragen. Zudem wird häufig eine unvollständige Lösung/Aufquellung der pulverförmigen Produkte beobachtet, was zu einer Qualitäts- und Stabilitätsverminderung der Endformulierung führt (Klumpen- und Quaddelbildung). Des weiteren sind bei der Verarbeitung bzw. Verwendung der pulverförmigen Produkte im allgemeinen besondere Rühr- und Dispergiervorrichtungen erforderlich, um die Polymere auf Basis von Acryloyldimethyltaurinsäure bzw. deren Salzen in den Mitteln zu lösen, bzw. zu suspendieren.

Aufgabe der vorliegenden Erfindung war die Entwicklung eines Eintopfverfahrens zur Herstellung von Polymerkonzentraten, enthaltend Copolymere auf Basis von Acryloyldimethyltaurinsäure bzw. deren Salzen. Die Polymerkonzentrate sollten bei Vorliegen in hochkonzentrierter flüssiger oder flüssig-disperser Form, d.h. bei Auftreten eines möglichst hohen Polymeranteils, eine niedrige Viskosität bei gleichzeitig hoher Stabilität der Lösung bzw. Dispersion aufweisen.

Überraschenderweise wurde gefunden, dass lagerstabile und thermostabile Konzentrate aus nachfolgend beschriebenen Copolymeren auf Basis von Acryloyldimethyltaurinsäure bzw. deren Salzen hergestellt werden können, indem man im Anschluss an die Polymerisationsreaktion ein Lösungsmittel zusetzt, dessen Siedepunkt höher ist, als der Siedepunkt des zur Polymerisation eingesetzten Polymerisationsmediums, Lösungsmittels bzw. Lösungsmittelgemischs und anschließend das niedriger siedende Polymerisationsmedium, Lösungsmittel bzw. Lösungsmittelgemisch, gegebenenfalls bei einem Druck, der im Vergleich zu Atmosphärendruck erniedrigt ist und gegebenenfalls bei einer Temperatur, die im Vergleich zu Raumtemperatur (25°C) erhöht ist, entfernt.

Gegenstand der Erfindung ist ein Verfahren zur Herstellung von Konzentraten in flüssiger oder flüssig-disperser Form, enthaltend
I) 10 bis 80 Gew.-%, bevorzugt 20 bis 60 Gew.-%, besonders bevorzugt 30 bis 40 Gew.-% eines Copolymeren enthaltend
   a) 1 bis 50 Gew.-% der wiederkehrenden Struktureinheit der Formel (1) 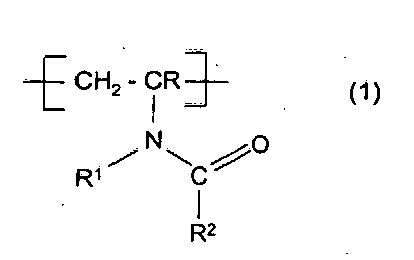 wobei R, R¹ und R² gleich oder verschieden sein können und Wasserstoff, eine lineare oder verzweigte Alkylgruppe mit jeweils 1 bis 30, bevorzugt 1 bis 20, insbesondere 1 bis 12 C-Atomen oder eine lineare oder verzweigte Alkenylgruppe mit jeweils 2 bis 30, bevorzugt 2 bis 20, insbesondere 2 bis 12 C-Atomen bedeuten, oder R¹ und R² zusammen eine C₂-C₉-Alkylengruppe bedeuten,
   b) 49,99 bis 98,99 Gew.-% der wiederkehrenden Struktureinheit der Formel (2) 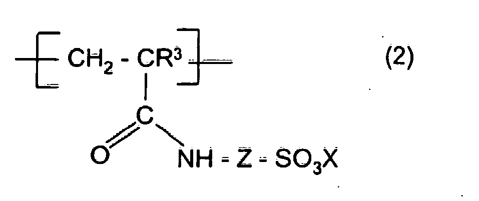 worin R³ Wasserstoff, Methyl oder Ethyl, Z C₁-C₈-Alkylen und X Ammonium-, Alkali- oder Erdalkali-Ion bedeutet, sowie
   c) 0,01 bis 8 Gew.-%, vorzugsweise 0,01 bis 5 Gew.-% vernetzende Strukturen, die aus Monomeren mit mindestens zwei olefinischen Doppelbindungen hervorgegangen sind,
II) 20 bis 90 Gew.-%, bevorzugt 30 bis 80 Gew.-%, besonders bevorzugt 40 bis 60 Gew.-% eines oder mehrerer Emulgatoren und/oder eines Lösungsmittels oder Lösungsmittelgemischs und
III) 0 bis 30 Gew.-%, bevorzugt 0 bis 10 Gew.-%, besonders bevorzugt 0 bis 5 Gew.-% Wasser,
dadurch gekennzeichnet, dass das Konzentrat hergestellt wird durch
a) radikalische Copolymerisation der Komponenten a), b) und c), vorzugsweise durch Lösungspolymerisation, Gelpolymerisation, nach einem Emulsionsverfahren, Fällungsverfahren, Hochdruckverfahren oder Suspensionsverfahren in einem Polymerisationsmedium, das sich bezüglich radikalischer Polymerisationsreaktionen weitestgehend inert verhält und die Bildung hoher Molekulargewichte zulässt, bevorzugt Wasser und niedere, tertiäre Alkohole oder Kohlenwasserstoffe mit 3 bis 30 C-Atomen, besonders bevorzugt tert.-Butanol,
b) dem Gemisch aus Polymer und Polymerisationsmedium ein höhersiedendes Lösungsmittel bzw. Lösungsmittelgemisch und/oder ein oder mehrere Emulgatoren und gegebenenfalls Wasser zugesetzt wird, wobei der Siedepunkt des höhersiedenden Lösungsmittels bzw. Lösungsmittelgemisches mindestens 10°C höher liegt als der des zur Polymerisation eingesetzten Polymerisationsmediums und
c) das niedriger siedende Polymerisationsmedium, gegebenenfalls bei einem Druck, der im Vergleich zu Atmosphärendruck erniedrigt ist und gegebenenfalls bei einer Temperatur, die im Vergleich zu Raumtemperatur erhöht ist, entfernt wird.

In einer bevorzugten Ausführungsform enthalten die nach dem erfindungsgemäßen Verfahren hergestellten flüssigen oder flüssig-dispersen Konzentrate Copolymere, die im wesentlichen aus den Struktureinheiten a), b) und c) bestehen.

Bevorzugt enthalten die nach dem erfindungsgemäßen Verfahren hergestellten flüssigen oder flüssig-dispersen Konzentrate Copolymere, bestehend aus 2 bis 30, insbesondere 3 bis 15 Gew.-% an Struktureinheiten der allgemeinen Formel (1), vorzugsweise abgeleitet von N-Vinylpyrrolidon, 69,5 bis 97,5 Gew.-%, insbesondere 84,5 bis 96,5 Gew.-% an Struktureinheiten der allgemeinen Formel (2), vorzugsweise abgeleitet vom Ammoniumsalz der 2-Acrylamido-2-methyl-propansulfonsäure und 0,2 bis 3, insbesondere 0,5 bis 2 Gew.-% an vernetzenden Strukturen, die aus Monomeren mit mindestens zwei olefinischen Doppelbindungen hervorgegangen sind. Die Copolymere können auch Mischungen verschiedener Struktureinheiten innerhalb der Formel (1) enthalten, vorzugsweise Mischungen aus Monomeren mit cyclischen und offenen Carbonamid-Gruppen. Das Mischungsverhältnis kann dabei innerhalb beliebiger Grenzen variieren.

Vernetzende Strukturen, die aus Monomeren mit mindestens zwei olefinischen Doppelbindungen hervorgegangen sind, leiten sich vorzugsweise ab von Acryl- oder Methacrylsäureallylester, Dipropylenglykoldiallylether, Polyglykoldiallylether, Triethylenglykoldivinylether, Hydrochinondiallylether, Tetraallyloxyethan oder anderen Allyl- oder Vinylethern multifunktioneller Alkohole, Tetraethylenglykoldiacrylat. Triallylamin, Trimethylolpropandiallylether, Methylen-bis-acrylamid oder Divinylbenzol.

Weiterhin bevorzugt leiten sich die vernetzenden Strukturen ab von Monomeren der allgemeinen Formel (3a). 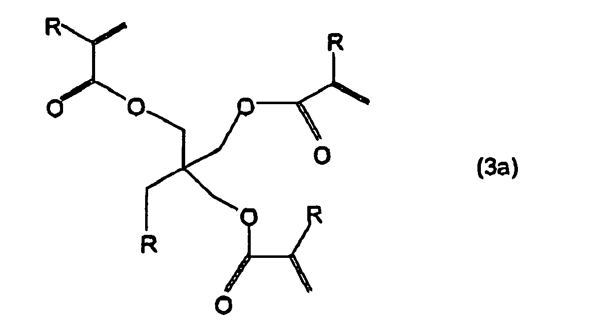 worin R Wasserstoff, Methyl oder Ethyl bedeutet.

In einer besonders bevorzugten Ausführungsform der Erfindung weist das Copolymer vernetzende Strukturen auf, die sich aus Monomeren der allgemeinen Formel (3) ableiten, 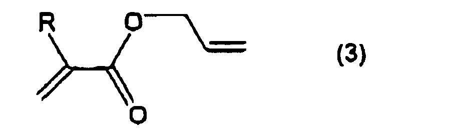 worin R Wasserstoff, Methyl oder Ethyl bedeutet.

Die Herstellung der Copolymere, die den nach dem erfindungsgemäßen Verfahren hergestellten Dispersionskonzentraten zugrunde liegen, erfolgt wie in EP 1 116 733 und EP 1 028 129 beschrieben, indem man die den wiederkehrenden Struktureinheiten der Formeln (1) und (2) entsprechenden Monomere in einem protischen Lösungsmittel löst oder dispergiert, zu dieser Lösung oder Dispersion einen oder mehrere Vernetzer mit mindestens zwei olefinischen Doppelbindungen zugibt und die Polymerisation in an sich bekannter Weise, z.B. durch Zugabe einer radikalbildenden Verbindung, startet.

In einer bevorzugten Ausführungsform wird die Polymerisation als Fällungspolymerisation durchgeführt.

Bei den Acryloyldimethyltauraten kann es sich um die anorganischen oder organischen Salze der Acryloyldimethyltaurinsäure (Acrylamidopropyl-2-methyl-2-sulfonsäure) handeln. Bevorzugt werden die Li⁺-, Na⁺-, K⁺-, Mg⁺⁺-, Ca⁺⁺-, Al⁺⁺⁺- und/oder NH₄⁺-Salze verwendet.

Der Neutralisationsgrad der Acryloyldimethyltaurinsäure kann zwischen 0 und 100 % betragen, besonders bevorzugt ist ein Neutralisationsgrad von oberhalb 80 %.

Die nach dem erfindungsgemäßen Verfahren hergestellten Konzentrate bzw. Dispersionskonzentrate enthalten neben dem Copolymer noch einen oder mehrere Emulgatoren und/oder ein Lösungsmittel bzw. Lösungsmittelgemisch in der angegebenen Menge. Bei Verwendung von Emulgatoren als alleiniger Komponente II) ist der Anteil des Lösungsmittels bzw. Lösungsmittelgemischs somit 0 % und entsprechend ist der Anteil der Emulgatoren 0 %, wenn die Komponente II) nur aus einem Lösungsmittel bzw. Lösungsmittelgemisch besteht. Bevorzugt verwendet man als zweite Komponente eine Mischung aus Emulgator und Lösungsmittel bzw. Lösungsmittelgemisch.

Als Emulgatoren kommen in Betracht Anlagerungsprodukte von 0 bis 30 Mol Alkylenoxid, insbesondere Ethylen-, Propylen-, Butylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitanester; (C₁₂-C₁₈)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und gegebenenfalls deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z.B. Polyglycerinpolyricinoleat und Polyglycerinpoly-12-hydroxystearat. Bevorzugt sind flüssige Fettsäureester, die sowohl ethoxyliert (PEG-10 Polyglyceryl-2 Laurate) als auch nicht ethoxyliert (Polyglyceryl-2 Sesquiisostearate) sein können.

Bevorzugte Emulgatoren sind Sorbitolester, hergestellt durch Reaktion von Sorbitol mit Fettsäuremethylestern oder Fettsäuretriglyceriden. Der Fettsäurerest in den Fettsäuremethylestern und Fettsäuretriglyceriden enthält im allgemeinen 8 bis 22 C-Atome und kann geradkettig oder verzweigt, gesättigt oder ungesättigt sein.

Beispiele hierfür sind Palmitinsäure, Stearinsäure, Laurinsäure, Linolsäure, Linolensäure, Isostearinsäure oder Ölsäure. Als Fettsäuretriglyceride kommen alle nativen tierischen oder pflanzlichen Öle, Fette und Wachse in Frage, beispielsweise Olivenöl, Rapsöl, Palmkernöl, Sonnenblumenöl, Kokosöl, Leinöl, Ricinusöl, Sojabohnenöl, gegebenenfalls auch in raffinierter oder hydrierter Form. Da diese natürlichen Fette, Öle und Wachse normalerweise Mischungen von Fettsäuren mit unterschiedlicher Kettenlänge darstellen, gilt dies auch für die Fettsäurereste in den erfindungsgemäß eingesetzten Sorbitolestern. Die erfindungsgemäß eingesetzten Sorbitolester können auch alkoxyliert sein, vorzugsweise ethoxyliert.

Des weiteren können anionische Emulgatoren, wie ethoxylierte und nicht ethoxylierte mono-, di- oder tri-Phosphorsäureester, aber auch kationische Emulgatoren wie mono-, di- und tri-Alkylquats und deren polymere Derivate eingesetzt werden.

Ebenfalls geeignet sind Gemische von Verbindungen aus mehreren dieser Substanzklassen.

Die nach dem erfindungsgemäßen Verfahren hergestellten Konzentrate können neben dem Polymer auf Basis von Acryloyldimethyltaurinsäure ein oder mehrere Lösungsmittel enthalten, bevorzugt ausgewählt aus der Gruppe der Kohlenwasserstoffe, Esteröle, pflanzlichen Öle und Silikonöle.
Die erfindungsgemäß eingesetzten Lösungsmittel umfassen Öle wie Kohlenwasserstofföle mit linearen oder verzweigten, gesättigten oder ungesättigten C₇-C₄₀-Kohlenstoffketten, beispielsweise Vaseline, Dodecan, Isododecan, Cholesterol, Lanolin, hydrierte Polyisobutylene, Docosane, Hexadecan, Isohexadecan, Paraffine und Isoparaffine;
Öle pflanzlichen Ursprungs, insbesondere flüssige Triglyceride wie Sonnenblumen-, Mais-, Soja-, Reis-, Jojoba-, Babusscu-, Kürbis-, Traubenkern-, Sesam-, Walnuss-, Aprikosen-, Makadamia-, Avocado-, Süßmandel-, Wiesenschaumkraut-, Ricinusöl, Olivenöl, Erdnussöl, Rapsöl und Kokosnussöl;
Öle tierischen Ursprungs, vorzugsweise Rindertalg, Perhydrosqualen, Lanolin; synthetische Öle wie Purcellinöl, lineare und/oder verzweigte Fettalkohole und Fettsäureester, bevorzugt Guerbetalkohole mit 6 bis 18, vorzugsweise 8 bis 10, Kohlenstoffatomen; Ester von linearen (C₆-C₁₃)-Fettsäuren mit linearen (C₆-C₂₀)-Fettalkoholen; Ester von verzweigten (C₆-C₁₃)-Carbonsäuren mit linearen (C₆-C₂₀)-Fettalkoholen, Ester von linearen (C₆-C₁₈)-Fettsäuren mit verzweigten Alkoholen, insbesondere 2-Ethylhexanol; Ester von linearen und/oder verzweigten Fettsäuren mit mehrwertigen Alkoholen (wie z.B. Dimerdiol oder Trimerdiol) und/oder Guerbetalkoholen; Alkoholester von C₁-C₁₀-Carbonsäuren oder C₂-C₃₀₋Dicarbonsäuren, C₁-C₃₀-Carbonsäuremonoester und Polyester von Zucker, C₁-C₃₀₋Monoester und Polyester von Glycerin;
Wachse wie Bienenwachs, Paraffinwachs oder Mikrowachse, gegebenenfalls in Kombination mit hydrophilen Wachsen, wie z.B. Cetylstearylalkohol; fluorierte und perfluorierte Öle;
Monoglyceride von C₁-C₃₀-Carbonsäuren, Diglyceride von C₁-C₃₀-Carbonsäuren, Triglyceride von C₁-C₃₀-Carbonsäuren, beispielsweise Triglyceride der Capryl/Caprinsäuren, Ethylenglykolmonoester von C₁-C₃₀-Carbonsäuren, Ethylenglycoldiester von C₁-C₃₀-Carbonsäuren, Propylenglycolmonoester von C₁-C₃₀-Carbonsäuren, Propylenglycoldiester von C₁-C₃₀-Carbonsäuren, sowie propoxllierte und ethoxilierte Derivate der oben genannten Verbindungsklassen.

Die nach dem erfindungsgemäßen Verfahren hergestellten Konzentrate können zusätzlich auch 0 bis 30 Gew.-%, bevorzugt 0 bis 10 Gew.-%, besonders bevorzugt 0 bis 5 Gew.-% Wasser enthalten.

Die Konzentrate eignen sich als Verdicker, Konsistenzgeber, Emulgator, Solubilisator, Dispergator, Gleitmittel, Haftmittel, Conditioner und/oder Stabilisator- hervorragend zur Formulierung von kosmetischen, pharmazeutischen und dermatologischen Mitteln, insbesondere von Öl-in-Wasser Emulsionen in Form von Cremes, Lotionen, Reinigungsmilch, Cremegele, Sprühemulsionen, z.B. Bodylotions, After-Sun Lotions, Sonnenschutzmittel und Deo-Sprays.

Der Vorteil dieser Konzentrate liegt darin, dass die oben definierten Copolymere In einer Darreichungsform vorliegen, die eine leichte Herstellung von kosmetischen, pharmazeutischen und dermatologischen Zubereitungen auf der Basis dieser Copolymere ermöglichen. Die Konzentrate sind überraschenderweise trotz ihres hohen Anteils an Copolymer gießfähig und lagerstabil.

Die Konzentrate werden in den kosmetischen, pharmazeutischen und dermatologischen Zubereitungen in solchen Gewichtsmengen eingesetzt, dass Polymerkonzentrationen von 0,01 bis 10 Gew.-%, bevorzugt 0,1 bis 5 Gew.-%, besonders bevorzugt 0,5 bis 3 Gew.-%, bezogen auf die fertigen Mittel resultieren.

Solche Zubereitungen können anionische, kationische, nichtionische, zwitterionische und/oder amphotere Tenside, sowie weitere Hilfs- und Zusatzstoffe, kationische Polymere, Filmbildner, Überfettungsmittel, Stabilisatoren, biogene Wirkstoffe, Glycerin, Konservierungsmittel, Perlglanzmittel, Farb- und Duftstoffe, Lösungsmittel, Trübungsmittel, ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen enthalten. Des Weiteren können den Mitteln antimikrobiell wirkende Agentien zugesetzt werden.

Zusätzlich können derartige Zubereitungen organische Lösungsmittel enthalten. Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, tert.-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt. Weitere geeignete Lösungsmittel sind beispielsweise Triacetin (Glycerintriacetat) und 1-Methoxy-2-propanol. Hydrotrop wirken kurzkettige Aniontenside, insbesondere Arylsulfonate, beispielsweise Cumol- oder Toluolsulfonat.

Die nachfolgenden Beispiele von Konzentraten mit Polymeren auf Basis von Acryloyldimethyltaurinsäure bzw. deren Salzen sollen den Gegenstand der Erfindung näher erläutern, ohne ihn darauf einzuschränken. Bei den Prozentangaben handelt es sich um Gew.-%.

### Beispiel A (aus Tabelle) (hohe Emulgatorkonzentration)

In einem 1-Liter-Planschliffkloben mit Temperaturfühler, Rückflusskühler, KPG-Rührer und pH-Kontrolle werden 500 g tert.-Butanol, 80 g Acryloyldimethyltaurinsäure bei 30°C vorgelegt. Anschließend wird durch Einleitung von gasförmigem Ammoniak neutralisiert und 5 g N-Vinylpyrrolidon und 2,0 g Trimethylolpropantriacrylat (Vernetzer) zu der Reaktionsmischung zugegeben. Die Reaktionsmischung wird anschließend durch Einleiten von N₂ inertisiert, auf 60°C aufgeheizt und die Reaktion nach 30 Minuten durch Zugabe von 2 g Dilauroylperoxid gestartet. Es kommt zu einer exothermen Reaktion, bei der die Innentemperatur um mehrere Grad ansteigt. Nach etwa 10 Minuten kommt es zur Ausfällung des entstehenden Polymers, was sich in einem ständigen Anstieg der Lösungsviskosität bemerkbar macht. Nach Beendigung der exothermen Phase (etwa 20-30 Minuten) wird die Reaktionsmischung zur Siedehitze erhitzt und 2 Stunden zur Vervollständigung der Reaktion nachgekocht. Dabei sinkt die Viskosität der Lösung wieder ab. Danach wird der Rückflusskühler durch eine Destillationsbrücke ersetzt. Nun werden 90 g Hostaphat KL 340D, 75 g Emulsogen SRO, 20 g Mineralöl (niedrig-viskos) und 20 g Isopropylpalmitat zu der Polymersuspension zugegeben und die Hauptmenge an tert.-Butanol anschließend unter gutem Rühren destillativ entfernt. Durch Anlegen eines Vakuums werden die Reste des tert.-Butanols aus der Mischung entfernt. Es ist darauf zu achten, dass das angelegte Vakuum zwar die destillative Abtrennung des tert.-Butanols ermöglicht, die bei diesem Druck korrespondierende Siedetemperatur des Lösemittels allerdings nicht überschreitet.

Nach erfolgter Abtrennung des tert.-Butanols wird abgekühlt und das Produkt aus dem Kolben ausgetragen.

### Beispiel G (aus Tabelle) (niedrige Emulgatorkonzentration)

In einem 1-Liter-Planschliffkloben mit Temperaturfühler, Rückflusskühler, KPG-Rührer und pH-Kontrolle werden 400 g tert.-Butanol und 80 g Acryloyldimethyltaurinsäure bei 30°C vorgelegt. Anschließend wird durch Einleitung von gasförmigem Ammoniak neutralisiert und 15 g N-Vinylformamid und 1,65 g TMPTA (Trimethylolpropantriacrylat) zu der Reaktionsmischung zugegeben. Die Reaktionsmischung wird anschließend durch Einleiten von N₂ inertisiert, auf 60°C aufgeheizt und die Reaktion nach 30 Minuten durch Zugabe von 1 g Dilauroylperoxid gestartet. Es kommt zu einer exothermen Reaktion, bei der die Innentemperatur um mehrere Grad ansteigt. Nach etwa 10 Minuten kommt es zur Ausfällung des entstehenden Polymers, was sich in einem ständigen Anstieg der Lösungsviskosität bemerkbar macht. Nach Beendigung der exothermen Phase (etwa 20-30 Minuten) wird die Reaktionsmischung zur Siedehitze erhitzt und 2 Stunden zur Vervollständigung der Reaktion nachgekocht. Dabei sinkt die Viskosität der Lösung wieder ab. Danach wird der Rückflusskühler durch eine Destillationsbrücke ersetzt. Nun werden 7,5 g Hostacerin DGI, 5 g Hostaphat KL 340D, 73 g Mineralöl (niedrig-viskos) und 73 g Isopropylpalmitat zu der Polymersuspension hinzugegeben und anschließend die Hauptmenge an tert.-Butanol unter gutem Rühren destillativ entfernt. Durch Anlegen eines Vakuums werden die Reste des tert.-Butanols aus der Mischung entfernt. Es ist darauf zu achten, dass das angelegte Vakuum zwar die destillative Abtrennung des tert.-Butanols ermöglicht, die bei diesem Druck korrespondierende Siedetemperatur des Lösemittels allerdings nicht überschreitet. Nach erfolgter Abtrennung des tert.-Butanols wird abgekühlt und das Produkt aus dem Kolben ausgetragen.

Es wurden verschiedene Konzentrate mit unterschiedlichen Emulgator- und Ölkonzentrationen hergestellt.

Tabelle 1 zeigt Beispiele von Konzentraten, die fließfähig und lagerstabil (Sedimentierung bei Lagerung 25°C; 3 Wochen) sind.

### Austauschseite 12

**Tabelle 1**

| Dispersionskonzentrat | A | B | C | D | E | F | G | H |
|---|---|---|---|---|---|---|---|---|
| Menge an Polymer | 36 | 36 | 36 | 30 | 36 | 36 | 36 | 30 |
| Hostacerin DGI | - | 30 | 3 | 51 | - | 30 | 3 | 51 |
| Hostaphat KL 340 D | 18 | 18 | 2 | 13 | 18 | 18 | 2 | 13 |
| Emulsogen SRO | 30 | - | - | - | 30 | - | - | - |
| Mineral Öl, niedrig viskos | 8 | - | 29,5 | 6 | 8 | - | 29,5 | 6 |
| Isopropyl Palmitate | 8 | - | 29,5 | - | 8 | - | 29,5 | - |
| Myritol 318 | - | 16 | - | - | - | 16 | - | - |

Die Zahlenangaben in Tabelle 1 bedeuten Gew.-%. Die Konzentrate B, D, E, F und H wurden analog zu A und das Konzentrat C wurde analog zu G hergestellt, wobei jedoch die Emulgatoren und Öle variiert wurden.

| | INCI-Name |
|---|---|
| Hostacerin DGI | Polyglyceryl-2-Sesquilsostearate |
| Hostaphat KL 340 D | Trilaureth-4 Phosphate |
| Emulsogen SRO | Rapeseed Oil Sorbitol Esters |
| | Mineral Öl, niedrig viskos |
| | Isopropyl Palmitate |
| Myritol 318 | Caprylic/Capric Triglyceride |

Beispiele zur Verwendung der Konzentrate bei der Herstellung kosmetischer Präparate. Die Prozentangaben bedeuten Gew.-%.

### Beispiel 1: Feuchtigkeitsspendende Lotion

| | | |
|---|---|---|
| A | Almondöl | 7,00 % |
| | Cyclomethicone | 5,00 % |
| B | Dispersionskonzentrat C | 4,00 % |
| C | Glycerin | 7,00 % |
| | Water | ad 100 % |
| | Konservierungsmittel | q.s. |
| D | Duftstoff | 0.30 % |

### Herstellung

- I: A und B mischen.
- II: Lösung von C in I einrühren.
- III: D zu II geben.
- IV: homogenisieren
- V: pH 5,5

### Beispiel 2: Sonnenschutzlotion

| | | |
|---|---|---|
| A | Vaselin | 5,00% |
| | Paraffinöl | 10,00% |
| | Dispersionskonzentrat A | 2,00 % |
| | Tocopherylacetat | 1,00 % |
| | Octylmethoxycinnamat | 2,00 % |
| | Parasol 1789 | 0,20 % |
| B | Ethanol | 10,00 % |
| C | Butylenglycol | 5,00 % |
| | Wasser | ad 100 % |

### Herstellung

- I: A und C werden getrennt auf 75°C erwärmt, danach vereinigt und unter Rühren auf 65°C abgekühlt, homogenisiert und weiter auf 35°C abgekühlt,
- II: B in I eingerühren, homogenisieren und auf Raumtemperatur abkühlen

### Beispiel 3: O/W - Hautmilch

| | | |
|---|---|---|
| A | Isopropylpalmitat | 4,00 % |
| | Mandelöl 5,00 % | 4,00 % |
| | Weizenkeimöl | 1,00 % |
| | ® Cetiol SN (Henkel) Cetearylisononanoat | 8,00 % |
| B | Dispersionskonzentrat G | 1,50 % |
| C | Wasser | ad 100 % |
| D | Duftstoffe | 0,30 % |

### Herstellung

- I: B unter Rühren zu A hinzugeben
- II: C und D zu I hinzurühren
- III: Emulsion homogenisieren

## Patentansprüche

1. Verfahren zur Herstellung von Konzentraten in flüssiger oder flüssig-disperser Form enthaltend
I) 10 bis 80 Gew.-% eines Copolymeren enthaltend
a) 1 bis 50 Gew.-% der wiederkehrenden Struktureinheit der Formel (1) 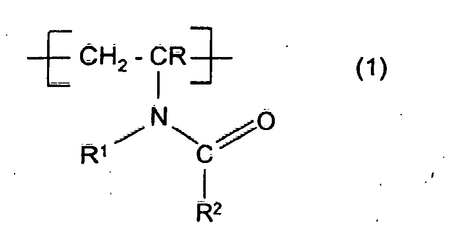 wobei R, R¹ und R² gleich oder verschieden sein können und Wasserstoff, eine lineare oder verzweigte Alkylgruppe mit jeweils 1 bis 30 C-Atomen oder eine lineare oder verzweigte Alkenylgruppe mit jeweils 2 bis 30 C-Atomen bedeuten, oder R¹ und R² zusammen eine C₂-C₉-Alkylengruppe bedeuten,
b) 49,99 bis 98,99 Gew.-% der wiederkehrenden Struktureinheit der Formel (2) 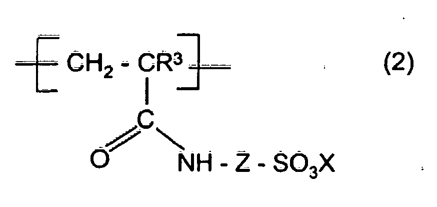 worin R³ Wasserstoff, Methyl oder Ethyl, Z C₁-C₈-Alkylen und X Ammonium-, Alkali- oder Erdalkali-Ion bedeutet, sowie
c) 0,01 bis 8 Gew.-% vernetzende Strukturen, die aus Monomeren mit mindestens zwei olefinischen Doppelbindungen hervorgegangen sind,
II) 20 bis 90 Gew.-% eines oder mehrerer Emulgatoren und/oder eines Lösungsmittels oder Lösungsmittelgemischs, sowie
III) 0 bis 30 Gew.-% Wasser,
**dadurch gekennzeichnet, dass** das Konzentrat hergestellt wird durch
a) radikalische Copolymerisation der Komponenten a), b) und c) in einem Polymerisationsmedium, das sich bezüglich radikalischer Polymerisationsreaktionen weitestgehend inert verhält und die Bildung hoher Molekulargewichte zulässt,
b) dem Gemisch aus Polymer und Polymerisationsmedium ein höhersiedendes Lösungsmittel bzw. Lösungsmittelgemisch und/oder ein oder mehrere Emulgatoren und gegebenenfalls Wasser zugesetzt wird, wobei der Siedepunkt des höhersiedenden Lösungsmittels bzw. Lösungsmittelgemisches mindestens 10°C höher liegt als der des zur Polymerisation eingesetzten Polymerisationsmediums und
c) das niedriger siedende Polymerisationsmedium, gegebenenfalls bei einem Druck, der im Vergleich zu Atmosphärendruck erniedrigt ist und gegebenenfalls bei einer Temperatur, die im Vergleich zu Raumtemperatur erhöht ist, entfernt wird.

2. Verfahren nach Anspruch 1, **dadurch gekennzeichnet, dass** das Copolymer aus 2 bis 30 Gew.-% an Struktureinheiten der allgemeinen Formel (1), vorzugsweise abgeleitet von N-Vinylpyrrolidon, 69,5 bis 97,5 Gew.-% an Struktureinheiten der allgemeinen Formel (2), vorzugsweise abgeleitet vom Ammoniumsalz der 2-Acrylamido-2-methyl-propan-sulfonsäure und 0,2 bis 3 Gew.-% an vernetzenden Strukturen, die aus Monomeren mit mindestens zwei olefinischen Doppelbindungen hervorgegangen sind, besteht.

3. Verfahren nach Anspruch 1 oder 2, **dadurch gekennzeichnet, dass** das Copolymer vernetzende Strukturen aufweist, die aus Monomeren mit mindestens zwei olefinischen Doppelbindungen hervorgegangen sind und sich ableiten von Acryl- oder Methacrylsäureallylester, Dipropylenglykoldiallylether, Polyglykoldiallylether, Triethylenglykoldivinylether, Hydrochinondiallylether, Tetraallyloxyethan oder anderen Allyl- oder Vinylethern multifunktioneller Alkohole, Tetraethylenglykoldiacrylat, Triallylamin, Trimethylolpropandiallylether, Methylen-bis-acrylamid oder Divinylbenzol.

4. Verfahren nach einem oder mehreren der Ansprüche 1 bis 3, **dadurch gekennzeichnet, dass** das Copolymer vernetzende Strukturen aufweist, die sich aus Monomeren der allgemeinen Formel (3) ableiten, 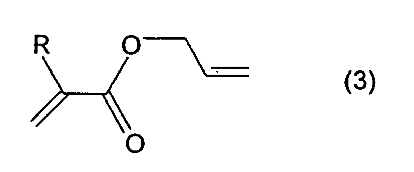 worin R Wasserstoff, Methyl oder Ethyl bedeutet.

5. Verfahren nach einem oder mehreren der Ansprüche 1 bis 4, **dadurch gekennzeichnet, dass** das Konzentrat 20 bis 60 Gew.-% an Copolymer enthält.

6. Verfahren nach einem oder mehreren der Ansprüche 1 bis 5, **dadurch gekennzeichnet, dass** das Konzentrat 30 bis 80 Gew.-% eines oder mehrerer Emulgatoren und/oder eines Lösungsmittels oder Lösungsmittelgemischs enthält.

7. Verfahren nach einem oder mehreren der Ansprüche 1 bis 6, **dadurch gekennzeichnet, dass** das Konzentrat 0 bis 10 Gew.-% Wasser enthält.

## Claims

1. A process for the preparation of concentrates in liquid or liquid-disperse form comprising
I) 10 to 80% by weight of a copolymer comprising
a) 1 to 50% by weight of the repeat structural unit of the formula (1) 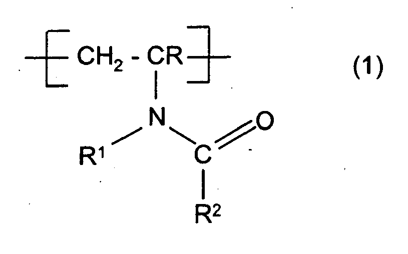 where R, R¹ and R² may be identical or different and are hydrogen, a linear or branched alkyl group having in each case 1 to 30 carbon atoms or a linear or branched alkenyl group having in each case 2 to 30 carbon atoms, or R¹ and R² together are a C₂-C₉-alkylene group,
b) 49.99 to 98.99% by weight of the repeat structural unit of the formula (2) 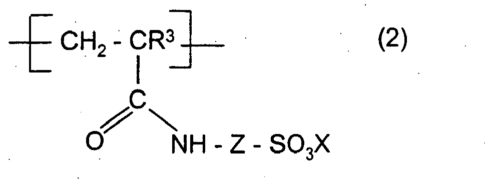 in which R³ is hydrogen, methyl or ethyl, Z is C₁-C₈-alkylene and X is an ammonium, alkali metal or alkaline earth metal ion, and
c) 0.01 to 8% by weight of crosslinking structures which have come from monomers with at least two olefinic double bonds,
II) 20 to 90% by weight of one or more emulsifiers and/or a solvent or solvent mixture, and
III) 0 to 30% by weight of water,
wherein the concentrate is prepared by
a) free-radical copolymerization of components a), b) and c) in a polymerization medium which behaves largely inertly with regard to free-radical polymerization reactions and permits the formation of high molecular weights,
b) a higher-boiling solvent or solvent mixture and/or one or more emulsifiers and optionally water is added to the mixture of polymer and polymerization medium, where the boiling point of the higher-boiling solvent or solvent mixture is at least 10°C higher than that of the polymerization medium used for the polymerization and
c) the lower-boiling polymerization medium is removed, optionally at a pressure which is lowered relative to atmospheric pressure, and optionally at a temperature which is increased relative to room temperature.

2. The process as claimed in claim 1, wherein the copolymer consists of 2 to 30% by weight of structural units of the formula (1), preferably derived from N-vinylpyrrolidone, 69.5 to 97.5% by weight of structural units of the formula (2), preferably derived from the ammonium salt of 2-acrylamido-2-methylpropanesulfonic acid and 0.2 to 3% by weight of crosslinking structures which have come from monomers with at least two olefinic double bonds.

3. The process as claimed in claim 1 or 2, wherein the copolymer has crosslinking structures which have come from monomers with at least two olefinic double bonds and are derived from acrylic or methacrylic allyl ester, dipropylene glycol diallyl ether, polyglycol diallyl ether, triethylene glycol divinyl ether, hydroquinone diallyl ether, tetraallyloxyethane or other allyl or vinyl ethers of multifunctional alcohols, tetraethylene glycol diacrylate, triallylamine, trimethylolpropane diallyl ether, methylenebisacrylamide or divinylbenzene.

4. The process as claimed in one or more of claims 1 to 3, wherein the copolymer has crosslinking structures derived from monomers of the formula (3), 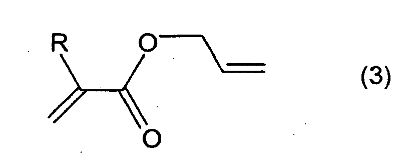 in which R is hydrogen, methyl or ethyl.

5. The process as claimed in one or more of claims 1 to 4, wherein the concentrate comprises 20 to 60% by weight of copolymer.

6. The process as claimed in one or more of claims 1 to 5, wherein the concentrate comprises 30 to 80% by weight of one or more emulsifiers and/or a solvent or solvent mixture.

7. The process as claimed in one or more of claims 1 to 6, wherein the concentrate comprises 0 to 10% by weight of water.

## Revendications

1. Procédé de préparation de concentrés sous une forme liquide ou liquide-dispersée, contenant
1) 10 à 80 % en poids d'un copolymère contenant
a) 1 à 50 % en poids du motif structural répétitif de formule (1) 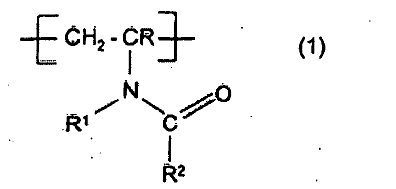 dans laquelle R, R¹ et R² peuvent être identiques ou différents et représentent chacun un atome d'hydrogène, un groupe alkyle à chaîne droite ou ramifiée ayant chacun 1 à 30 atomes de carbone, ou un groupe alcényle à chaîne droite ou ramifiée ayant chacun 2 à 30 atomes de carbone, ou bien R¹ et R² forment ensemble un groupe alkylène en C₂-C₉,
b) 49,99 à 98,99 % en poids du motif structural répétitif de formule (2) 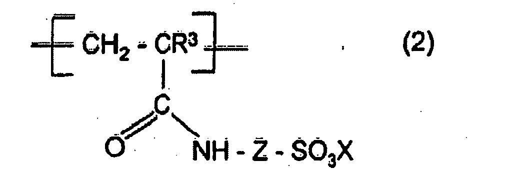 dans laquelle R³ est un atome d'hydrogène ou le groupe méthyle ou éthyle, Z est un radical alkylène en C₁-C₈, et X est un ion ammonium, métal alcalin ou métal alcalino-terreux, ainsi que
c) 0,01 à 8 % en poids de structures réticulables qui proviennent de monomères ayant au moins deux doubles liaisons oléfiniques,
II) 20 à 90 % en poids d'un ou plusieurs émulsifiants et/ou d'un solvant ou d'un mélange de solvants, et aussi
III) 0 à 30 % en poids d'eau,
**caractérisé en ce que** le concentré est préparé par
a) copolymérisation radicalaire des composants a), b) et c) dans un milieu de polymérisation qui présente un comportement presque parfaitement inerte par rapport à des réactions de polymérisation radicalaire, et qui autorise la formation de grandes masses moléculaires,
b) on ajoute au mélange du polymère et du milieu de polymérisation un solvant ou un mélange de solvants à haut point d'ébullition et/ou un ou plusieurs émulsifiants et éventuellement de l'eau, le point d'ébullition du solvant ou du mélange de solvants à haut point d'ébullition étant d'au moins 10°C supérieur à celui du milieu de polymérisation utilisé pour la polymérisation,
c) on élimine le milieu de réaction à bas point d'ébullition, éventuellement sous une pression plus faible que la pression atmosphérique, et éventuellement à une température supérieure à la température ambiante.

2. Procédé selon la revendication 1, **caractérisé en ce que** le copolymère est constitué de 2 à 30 % en poids de motifs structuraux de formule générale (1), qui dérivent de préférence de la N-vinylpyrrolidone, de 69,5 à 97,5 % en poids de motifs structuraux de formule générale (2), qui dérivent de préférence du sel d'ammonium de l'acide 2-acrylamido-2-méthyl-propanesulfonique, et de 0,2 à 3 % en poids de structures réticulables qui proviennent de monomères ayant au moins deux doubles liaisons oléfiniques.

3. Procédé selon la revendication 1 ou 2, **caractérisé en ce que** le copolymère comprend des structures réticulables qui proviennent de monomères ayant au moins deux doubles liaisons oléfiniques, et qui dérivent de l'acrylate ou du méthacrylate d'allyle, de l'éther diallylique du dipropylèneglycol, de l'éther diallylique du polyglycol, de l'éther divinylique du triéthylèneglycol, de l'éther diallylique de l'hydroquinone, du tétraallyloxyéthane ou d'autres éthers allyliques ou vinyliques de polyalcools, du diacrylate de tétraéthylèneglycol, de la triallylamine, de l'éther diallylique du triméthylolpropane, du méthylène-bis-acrylamide ou du divinylbenzène.

4. Procédé selon l'une ou plusieurs des revendications 1 à 3, **caractérisé en ce que** le copolymère comprend des structures réticulables qui dérivent de monomères de formule générale (3) 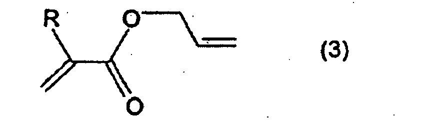 dans laquelle R est un atome d'hydrogène ou le groupe méthyle ou éthyle.

5. Procédé selon l'une ou plusieurs des revendications 1 à 4, **caractérisé en ce que** le concentré contient 20 à 60 % en poids d'un copolymère.

6. Procédé selon l'une ou plusieurs des revendications 1 à 5, **caractérisé en ce que** le concentré contient 30 à 80 % en poids d'un ou plusieurs émulsifiants et/ou d'un solvant ou d'un mélange de solvants.

7. Procédé selon l'une ou plusieurs des revendications 1 à 6, **caractérisé en ce que** le concentré contient 0 à 10 % en poids d'eau.
